**Europäisches Patentamt**

**European Patent Office**

**Office européen des brevets**

(19)

(11) Numéro de publication: **0 004 214**

**A1**

# (12) DEMANDE DE BREVET EUROPEEN

(21) Numéro de dépôt: **79400094.3**

(22) Date de dépôt: **15.02.79**

(51) Int. Cl.²: **A 61 K 37/54**
**//C12D13/10**

(30) Priorité: **16.02.78 CH 1719/78**

(43) Date de publication de la demande:
**19.09.79 Bulletin 79/19**

(84) Etats contractants désignés:
**BE FR GB IT NL SE**

(71) Demandeur: **SCIENCE UNION ET Cie SOCIETE**
**FRANCAISE DE RECHERCHE MEDICALE**
**14 rue du Val d'Or**
**F-92150 Suresnes(FR)**

(72) Inventeur: **Simon-Lavoine, Nicole**
**2 rue du Chateau**
**F-92200 Neuilly/Seine(FR)**

(72) Inventeur: **Forgeot, Marcel**
**26 rue Berthollet**
**F-75013 Paris(FR)**

(74) Mandataire: **Burtin, Jean-François**
**SCIENCE UNION ET Cie Service Brevets 22 rue Garnier**
**F-92200 Neuilly(FR)**

(54) **Nouvelles compositions pharmaceutiques à base d'enzyme bactérien.**

(57) L'invention se rapporte à de nouvelles compositions pharmaceutiques dont le principe actif est un enzyme obtenu à partir de cultures microbiennes.

Plus précisément l'invention se rapporte à des compositions pharmaceutiques destinées au traitement des maladies virales caractérisées en ce qu'elles renferment à titre de principe actif une N-acétylneuraminate glycohydrolase extraite de cultures de Streptococcus pneumoniae en association avec un véhicule ou un excipient inerte non toxique pharmaceutiquement acceptable. Ces compositions sont destinées à l'administration par voie parentérale, permuqueuse, perlinguale ou rectale.

Croydon Printing Company Ltd.

EP 0 004 214 A1

L'invention a pour objet de nouvelles compositions pharmaceutiques renfermant comme principe actif un composé enzymatique.

L'invention a plus précisément pour objet de nouvelles compositions pharmaceutiques destinées au traitement de maladies virales dont le principe actif est une glycoside hydrolase bactérienne.

L'invention a spécifiquement pour objet des compositions pharmaceutiques destinées au traitement des maladies virales, caractérisées en ce qu'elles renferment à titre de principe actif la N-acétylneuraminate glycohydrolase obtenue à partir des cultures de Streptococcus pneumoniae (famille des Micrococcaceae).

La N-acétylneuraminate glycohydrolase est un enzyme capable de libérer l'acide N-acétylneuraminique des glyco-protéines lorsqu'il occupe l'extrémité non réductrice de la chaîne d'un polysaccharide.

On connaît un certain nombre de N-acétylneuraminate glycohydrolases ou neuraminidases d'origine bactérienne ou virale.

C'est ainsi qu'on a déjà décrit les neuraminidases de Vibrion cholérique, de Clostridium perfringens, de Vibrion Comma, ou de Virus Influenzae divers.

A chacune de ces neuraminidases corresponde une structure glycoprotéique distincte et des propriétés physiques et chimiques différentes.

2

Dans le cas de la N-acétyheuraminate glycohydrolase extraite de Streptococcus pneumoniae, on a constaté que le produit obtenu selon les modalités décrites ci-après, possédait des caractéristiques physiques qui la différenciait des autres neuraminidases connues jusqu'à présent. La souche responsable d'une telle production est celle de Streptococcus pneumoniae I-044 ( Collection de l'Institut Pasteur de Paris ).

En effet cet enzyme ne possède aucune action galactosidasique, protéasique, N-acétyl glucosaminidasique, fucosidasique, ou arylsulfatasique au contraire des autres neuraminidases.

En outre, la constante de Michaelis en utilisant les glycopeptides bronchiques comme substrat est de $K_M = 2,3.\ 10^{-4}$ , ce qui montre une très forte affinité pour ce substrat.

Le point isoélectrique déterminé par isoélectrofocalisation montre deux pics d'activité de cet enzyme, traduisant ainsi l'existence de deux isoenzymes de pHi = 4,2 et 3,7. Ce fait la différencie de la neuraminidase isolée de Clostridium Perfringens qui est homogène et de point isoélectrique de 4,7.

L'enzyme de Streptococcus pneumoniae libère l'acide N-acétyl neuraminique de l'$\alpha_1$- glycoprotéine acide humaine à 82% en 7 heures et la quasi totalité (90%) de l'acide N-acétyl neuraminique d'une Sialomucine bronchique en 6 heures d'action.

La structure chimique de la N-acétylmuraminate glycohydrolase de Streptococcus pneumoniae a déjà été décrite par P. Bienvenu , C. Gaget, C. Bottex et R. Fontanges [C.R. Acad. Sci. (Paris) 285 ( section D) 837 (1977) ] ainsi que sa teneur en différents amino-acides. Elle établit son individualité par rapport à la Neuraminidase obtenue par hydrolyse enzymatique du Virus grippal A (Hong Kong ) 1/68.

Les compositions pharmaceutiques selon l'invention, se présentent sous une des formes convenant pour l'administration par voie parentérale, permuqueuse, percutanée, perlinguale ou rectale.

Elles se présentent notamment sous forme de solutions ou de suspensions injectables, réparties en ampoules, en flacons multidoses ou en seringues prêtes à l'injection, de comprimés sublinguaux, de préparations préssurisées pour la pulvérisation sur les muqueuses nasales ou pharyngées, de solutions dans un solvant pénétrant pour l'usage percutané, de comprimés sublinguaux et de suppositoires.

Les compositions pharmaceutiques selon l'invention renfermant en outre un ou plusieurs excipients ou véhicules inertes, non toxiques, pharmaceutiquement compatibles, adaptés à l'administration par voie parentérale, permuqueuse, percutanée, perlinguale ou rectale.

On pourra en outre leur adjoindre un ou des agents d'adhésion ou de remplissage, des diluants, des agents de dispersion, des agents tensio-actifs, des agents parfumants, des arômes, des agents émulsionnants ou des agents retardant leur diffusion dans l'organisme. Selon la voie d'adminsitration, la posologie unitaire peut varier dans de larges proportions. Elle s'échelonne notamment entre 300 U et 5000 U par unité de prise chez l'adulte.

La posologie journalière est en particulier comprise entre 1 et 4 fois la posologie unitaire chez l'adulte.

En particulier, dans les cas d'administration par voie permuqueuse, nasale ou buccale, on aura recours à 3 pulvérisations d'une solution ou suspension dans un véhicule aqueux pendant deux jours. Dans le cas d'administration par voie parentérale , on aura recours à un traitement à raison d'une injection par jour pendant une semaine puis une administration par semaine pendant deux mois.

4

Les compositions pharmaceutiques selon l'invention trouvent leur emploi dans le traitement des maladies virales et bactériennes. Elles agissent en particulier sur les Myxovirus Influenzae, Rhinovirus et toutes les lignées de virus avec neuraminidase. Cet effet peut être attribué soit à une action directe sur l'enveloppe glycoprotéique virale en diminuant le nombre de lésions cellulaires liées à la reproduction des éléments viraux. Elles peuvent également agir par un effet immunostimulant spécifique sur des germes dont l'action pathogène est médiée par une neuraminidase de structure antigénique voisine ( par exemple, Erysipelotrix chez les animaux ). Enfin, on peut leur attribuer un effet immunostimulant non spécifique qui complète leur emploi dans les rhinites, rhinopharyngites, sinusites, angines, amygdalites, laryngites, trachéites, bronchites aiguës, bronchiolites, bronchopneumonies ou bronchites chroniques.

On peut aussi leur attribuer une action antitumorale par élimination de l'acide sialique de la surface des membranes cellulaires ce qui augmente l'immunogénicité de celles-ci.

C'est ainsi que les compositions pharmaceutiques selon l'invention :

a) administrées préventivement, les 2 premiers jours du mois pendant 6 mois d'hiver chez le sujet exposé à la grippe, ou avant les symptômes chez l'entourage du sujet grippé, préviennent la phase de dissémination virale après infestation cellulaire, empêchent l'apparition des signes cliniques de l'infection grippale : fièvre, algie, fatigue, courbatures, maux de tête ... : préviennent les surinfections bactériennes.

b) administrées curativement, dès les premiers signes, augmentent la restauration cellulaire, quelle que soit la souche responsable, accélèrent la guérison clinique, stoppent les complications notamment pneumopathologiques.

Elles présentent le grand avantage du point de vue innocuité d'exister à l'état ultra-purifié grâce à des procédés très élaborés de fabrication, de posséder un pH neutre, d'être exemptes de

5

toxicité propre vis-à-vis de la cellule, d'être dépourvues des inconvénients inhérant aux vaccins fabriqués à partir de virus atténués ou tués, dont l'activité n'est que préventive, dont l'antigénicité est peu importante et la production d'anticorps faible; dont la toxicité est loin d'être négligeable; dont la pureté inté - grale n'est pas toujours assurée.

L'effet immunostimulant est bien vérifié expérimentalement:

In vitro l'activation des macrophages est attesté par :

. un plus grand étalement

. une augmentation de la phagocytose

. une augmentation du nombre de rosettes E et de rosettes EAC

. une augmentation de la synthèse de D.N.A. en présence de lectine.

In vivo on peut mettre en évidence

. l'augmentation de la réponse humorale

. une stimulation de l'hypersensibilité retardée.

On peut donc envisager leur emploi pour favoriser la production d'un auto vaccin à partir de cellules tumorales ou d'hétérovaccin.

En outre, par suite de son action sur les glycoprotéines du mucus bronchique, la Neuraminidase bactérienne produite par Streptococcus pneumoniae n° I-044 entraine une diminution importante de la viscosité pouvant aller jusqu'à 40 %.

Ce type d'action rend la Neuraminidase selon l'invention utile pour le traitement des affections bronchiques chroniques.

L'effet immunostimulant et antiviral des compositions selon l'invention paraît d'autant plus surprenant et imprévisible que les études expérimentales effectuées par injection intratumorale, notamment dans un carcinome malphigien, cf.P.Binder, C.R. Acad. Sci. 281 1975 série D, 1545) ont entrainé chez le rat une mortalité précoce très importante par dissémination des métastases. De même des cellules tumorales préalablement tuées par exposition aux Rayons X puis traitées par la Neuraminidase et injectées à des souris avant implantation de cellules tumorales B$_{16}$, ont stimulé la croissance des tumeurs implantées.

De même les travaux de P. Bienvenu et al. (loc.cit) ont montré que la Neuraminidase bactérienne apparait comme dépourvue de pouvoir protecteur contre l'infection par le virus grippal.

Pour toutes ces raisons les propriétés antivirales et immunostimulantes utilisables en thérapeutique humaine ou animale apparaissent comme inattendues au regard des publications antérieures et constituent la première introduction en thérapeutique des compositions pharmaceutiques contenant comme principe actif la N-acétylheuraminate glycohydrolase de Streptococcus pneumoniae.

Les exemples suivants illustrent l'invention . Ils ne la limitent en aucune façon.

EXEMPLE 1

Obtention de la N-acétyl neuraminate-glycohydrolase de Streptococcus pneumoniae.

I- Souche

La souche élaborant la neuraminidase est un Streptococcus appartenant à la famille des Micrococcaceae, n° I-044 de la collection de l'Institut Pasteur de Paris.

. Caractères

Il s'agit de Cocci gram positif, en paires, parfois très courtes chaînettes, (quelquefois) encapsulée à caractère aérobie facultatif. La température optimale de culture est voisine de 37°C.

. Entretien

Cette souche est entretenue sur gélose ( Institut Pasteur)

7

additionée d'acide nalidixique.

1) <u>Préparation de ce milieu</u>

On met en suspension 29g de milieu déshydraté dans 1 1. d'eau distillée, on attend 5 minutes, puis mélange jusqu'à obtention d'une suspension homogène. On fait chauffer lentement en agitant puis laisse bouillir jusqu'à complète dissolution. On ajuste le pH à 7,2, si nécessaire puis répartit en tubes puis stérilise à l'autoclave, 20mn à 120°C. Après stérilisation , on laisse refroidir jusqu'à 50°C, puis ajoute 10% de sang de cheval stérile. On homogénise doucement pour ne pas provoquer de bulles d'air, et laisse refroidir en position inclinée.

2) <u>Ensemencement</u>

L'ensemencemencement s'effectue en stries, à la surface de de la gélose inclinée. L'incubation de ces géloses ensemencées se fait en étuve à 37°C, pendant 18h. à 24h.. L'inoculat est ensuite lyophylisé pour la conservation sur lait écrémé reconstitué à 20%

II- <u>Préparation de l'inoculum</u>

On remet en suspension le contenu d'un tube lyophylisé, dans environ 2 ml. du milieu suivant :

| | | |
|---|---|---|
| . Brain-heart ( Difco) | 25 | g/l |
| . Néopeptone ( Difco) | 1 | g/l |
| . Eau distillée q.s.p. | 1 | 1 |
| . pH ajusté à | 7,8 avec de la soude | |
| . Stérilisation | 30' à 120°C | |
| . pH après stérilisation | 7,6 | |

On utilise cette suspension pour ensemencer un ballon contenant 4 1. du même milieu.

L'incubation anaérobie se fait à 37°C, en enceinte thermorégulée, sans agitation, ni aération, pendant 24 h.

8

III- Production

On transfère les 4 1. du ballon, pour ensemencer le fermenteur de production contenant 1 m$^3$ de milieu de composition suivante :

.  Pastose ( Institut Pasteur)          10,0  g/l
.  Cérébrine                             1,0  g/l
.  Extrait de viande ( Mérieux)          3,0  g/l
.  Autolysat de levure (Fould-Springer   3,0  g/l
.  Phosphate bipotassique                1,0  g/l
.  Chlorure de sodium                    5,0  g/l
.  Eau de ville q.s.p.                   1    1.
.pH spontané : 6,8 - 7,0 ajusté à 7,8 - 8,0 avec de la soude
.  Stérilisation                         20' à 120 °C
.  pH après stérilisation                8,0 - 8,2

Conditions de fermentation :
.  Aération        : néant
.  Agitation       : 20 t/mn
.  Température      : 37°C
.  Durée   :       : 29 h.

A la fin de la fermentation , ajouter 0,1 g/l de lysosyme pour favoriser l'éclatement des cellules et permettre la diffusion de la neuraminidase dans ce milieu. On agite pendant 3h. à 37°C. On laisse refroidir ensuite aux environs de 20°C. On suit la progression de la fermentation par :

- des examens microcospiques pour suivre le développement du Streptococcus pneumoniae

- des prises de pH, le milieu s'acidifiant légèrement pour atteindre environ 6,5 en fin de fermentation; des dosages de l' activité neuraminidasique.

IV - Extraction

Par précipitations fractionnées

1) <u>Addition de 500 g/l de sulfate d'ammonium</u> par litre de moût en présence de 3 g/l d'hyflosupercel. Agitation 1 h. Filtration sur filtre-presse. Lavage du gâteau par une solution aqueuse de sulfate d'ammonium à 40 %. Le gâteau recueilli est repris par de l'eau distillée à + 4°C : 3 fois 5 l.

2) <u>Filtrat 25 %</u>

Aux 15 l. de reprises obtenues en IV 1), on ajoute, après dosage des ions $SO_4$, la quantité nécessaire de sulfate d'ammonium pour obtenir une concentration de 250 g/l, en présence de 40 g/l d'hyflosupercel. Agitation : 1 h. Filtration sur Büchner.

3) <u>Gâteau 40 %</u>

Au filtrat obtenu en IV 2), on ajoute: 150 g/l de sulfate d'ammonium, en présence de 10 g/l d'hyflosupercel. Agitation 1 h. Filtration sur Büchner. Lavage du gâteau par une solution aqueuse de sulfate d'ammonium à 400 g/l. Les extraits sont réunis et dialysés contre un courant d'eau de ville, à + 4°C, pendant 15 h. à un débit de 20 l/h.

4) <u>Chromatographie sur Carboxyméthylcellulose CM23</u>

<u>Préparation de la colonne</u> : ( diamètre 15 cm - hauteur 100 cm). Sur la colonne contenant 1,6 Kg de carboxyméthylcellulose CM 23, on passe une solution tampon phosphate 0,0025 M (pH 6,8) 55 l.

<u>Fixation</u>

Les extraits dialysés sont clarifiés par filtration, puis ajustés à pH 6,8 avec de l'acide citrique $1_M$. On effectue un passage sur la colonne par du tampon phosphate 0,0025 M, (pH 6,8) avec un volume de 10 à 15 l.

. Volume mort     : 6 l.

. Volume des effluents + lavages  : 12 l.

10

Elution

La neuraminidase fixée est éluée par une solution tampon phosphate-citrate 0,2 M de pH 6,8 : de 10 l. à 15 l. sous un débit de 4 l/h.

. Volume mort                          : de 6 l. à 8 l.

. Volume d'un éluat "coeur"        :     5 l.

Le"coeur"est dialysé contre un courant de chlorure de sodium à 1%; sous un débit de 4 l/h. pendant 16 h. à + 4°C.

Après lyophilisation on obtient la neuraminidase brute d'activité spécifique voisine de 500.

Purification par passage sur Séphadex G 50

On percole, à travers une colonne de Séphadex G 50 (diamètre 6 cm - hauteur 90 cm ) équilibré contre un tampon phosphate - citrate 0,1 M de pH 6,8. On développe par le même tampon et recueille des fractions de 20 ml. Les fractions contenant la neuraminidase sont réunies, dialysées pendant 24 h. contre un courant de solution aqueuse de chlorure de sodium à 1 % puis lyophilisées. L'activité spécifique de la poudre obtenue est voisine de 20.000. Elle est mesurée par détermination de la quantité d'acide sialique libéré à 37° en une minute avec la NANL comme substrat, ramenée à 1 mg de protéine, et exprimée en Nanomoles ( NANL = N-acétyl Neuraminyl lactose).

Propriétés enzymatiques de la N-acétyl neuraminate glycohydrolase purifiée extraite des cultures de Streptococcus pneumoniae.

| Activité recherchée (par mg de protéine) | Préparation brute de Neuraminidase | Neuraminidase purifiée après passage sur carboxyméthyl cellulose | Neuraminidase purifiée après passage sur Sephadex G 50 |
|---|---|---|---|
| Neuraminidase | 3,1 | 500 | 20 000 |
| N. acétyl glucosaminidase | 1,6 | 0 | 0 |
| β-galactosidase | 0,3 | 0 | 0 |
| α-galactosidase | 0,03 | 0 | 0 |
| α-fucosidase | 0 | 0 | 0 |
| β-fucosidase | 0 | 0 | 0 |
| Arylsulfatase | 0 | 0 | 0 |
| Protéase | 0 | 0 | 0 |

EXEMPLE II

Absence de communauté antigénique entre la neuraminidase de Streptococcus pneumoniae et les neuraminidases d'origine virale.

a) les essais de neutralisation de l'activité de la neuraminidase de Streptococcus pneumoniae (souche n° I-044) par des sérums de lapins immunisés contre les neuraminidases d'origine virale ont été entièrement négatifs.

Virus essayés

- <u>Myxovirus influenzae</u>
  . <u>humains</u> : <u>type A</u> - $N_2$
                Singapour 57
                Hong Kong 68
                England   72
                <u>type B</u>
  . animaux : A equi 1
              A equi

- <u>Myxovirus para influenzae</u>
  . humains : type 1,2,3
              oreillons

  . animaux : sendaï SV 5

b) les essais de neutralisation des neuraminidases virales par un sérum de lapin anti-neuraminidase de Streptococcus pneumoniae sont restés négatifs.

EXEMPLE III

Effet de la neuraminidase bactérienne sur la multiplication du virus grippal A port Chalmers 1/73 $H_3N_2$.

Les différents lots de neuraminidase bactérienne ont été testés sur des fragments de coquilles d'oeufs + MCA maintenus en survie dans des cupules de plaques Perspex (technique des egg-bits de FAZEKAS). Ces deux sortes de cellules ont été inoculées avec du virus A, Port Chalmers (0,1 ml de dilution + 0,9 ml milieu pour un titre infectieurx du virus égal à $10^{-5}$ $ID_{50}$/ 0,1 ml). Après une incubation de 6 heures à 33°, on ajoute la neuraminidase bactérienne (0,1 ml dilution $10^{-2}$ pour un titre neuraminidasique spécifique de

13

8 000 et une concentration de 1 mg protéines /ml).

L'action de cet enzyme sur la multiplication du virus se fait pendant 72 heures. Au bout de ce temps, on détermine le titre infectieux du virus produit, par détermination du titre hemagglutinant HA ( sur egg bits ) au moyen de globules rouges de poule mis directement dans le milieu. Les résultats observés pour la neurami nidase bactérienne sont reportés dans le tableau I. Tous les essais ont été inoculés à la même dilution, ayant un même titre et une même concentration en protéines.

L'effet de la neuraminidase bactérienne sur la multiplication du virus a été testé en inoculant toutes les 6 heures la même doses de neuraminidase. Le virus se développe pendant les 6 premiè res heures sur les "egg-bits" maintenus en survie et incubés à 33°.

A la sixième heure, on ajoute une dose de neuraminidase bactérienne. On laisse la multiplication se faire jusqu'à 72 heures. Le titre infectieux est déterminé par hémagglutination avec des globules rouges de poule à 2%.

Le tableau I ci-après fournit la cinétique de multiplication du virus grippal en présence ou en l'absence de neuraminidase bactérienne. Il permet d'établir l'incidence considérable de la neuraminidase bactérienne de S. pneumoniae sur la multiplication du virus de la grippe.

EXEMPLE IV

Détermination de l'effet de la neuraminidase de Streptococ cus pneumoniae sur le taux de multiplication de Myxovirus (souches sauvages) selon le délai d'introduction.

1ère expérience :

La neuraminidase a été inoculée au cours de la multiplication des myxovirus en culture sur egg bits, à la dose de 2,7U / ml / cupule de réaction.

La multiplication virale, en absence de neuraminidase de S.pneumoniae est suivie pendant 72 h. à 33°. Au bout de ce temps, sur une partie de la culture, on effectue une hémagglutination, directement dans les cupules, avec des globules rouges de poule à 2% ( titre HA/MCA). L'autre partie est récoltée en pool, dosée pour ses activités hémagglutinante et neuraminidasique et son titre infectieux sur oeufs embryonnés ( EID 50) avant d'être ré-inoculée sur de nouveaux egg bits.

De la même façon, cette 2ème multiplication virale est suivies pendant 72h. à 33°, puis récoltée, dosée pour ses activités hémagglutinante et neuraminidasique et titre infectieux et à nouveau nouveau inoculée sur des egg bits frais.

Le schéma de la 3ème multiplication virale est identique aux deux précédentes.

Parallélement, on suit la multiplication des virus en présence de neuraminidase inoculée à la 6ème heure du cycle viral, pour chacune des 3 multiplications.

Le restant des pools, de chaque multiplication récoltée à 72h. subit une concentration, puis une purification afin d'observer la morphologie des virus produits en absence et en présence de neuraminidase.

Des egg bits recouverts d'un fragment de membrane chorio-allantoïque ( MCA ) sont prélevés en cours de multiplication au temps 26 h. pour chacun des trois passages. Certains sont fixés dans de la glutaraldéhyde à 2% dans le but d'observer au microscope électronique, la sortie du virus au niveau de la membrane. D'autres sont fixés dans la glutaraldéhyde à 1% dans le but de faire du cryodécapage et d'étudier les différences de morphologie des membranes infectées et non infectées, traitées et non traitées par la neuraminidase.

2ème expérience

La neuraminidase est inoculée au cours de la multiplication des myxovirus $A_2$ PC/73 et $A_2$ VIC/3/75 à la concentration de 0,5 U/ml/cupule de réaction.

$A_2$ VIC/3/75 de liquide allantoïque

caractéristiques: titre HA : 200
titre NA : 80
EID 50 : $10^{7,5}$
HA s/MCA : $10^{4,5}$

Neuraminidase bactérienne: activité spécifique : 27 000

Solution-mère à 0,1 mg prot/ml   2 7000 UN/ml
inoculation     0,1 ml dilué au 1/100   2,7 UN/ml/cupule Solution-mère diluée au 1/5        540 UN/ml
inoculation                    0,1 ml dilué au 1/100 0,54UN/ml/ cupule

On utilise les techniques habituelles de
. culture sur egg bits
. dosage d'hémagglutine
. dosage de neuraminidase
. détermination EID 50 sur oeufs embryonnés.

On procède ensuite à la fixation des membranes pour microscopie électronique et cryodécapage.

Résultats

Les résultats obtenus sont rassemblés dans les histogrammes I, II, III suivants :

Il en ressort que par addition de la neuraminidase de S. pneumoniae 6 h. et 24 h. après la mise en culture du virus grippal sur fragment de coquille d'oeuf, le taux de multiplication du virus est inférieur d'au moins 2 log 10 à celui des témoins. L'administration après 48 h. et 54 h. produit un effet faible mais qui n'est pas nul.

EXEMPLE V

Etude du taux de multiplication des virus grippaux en présence de quantités variables de neuraminidase de S.pneumoniae.

On établit une gamme de solution de neuraminidase bactérienne de différentes concentrations protéiques:

0,1 - 0,2 - 0,4 - 0,6 - 0,8 - 1 mg de protéines/ml

On inocule une dose (0,1 ml $10^2$) de ces différentes solutions à des cultures sur MCA ( technique des egg bits) de différents virus grippaux, pour obtenir leur effet sur la multiplication des virus.

Matériel et Méthodes

- virus grippaux influenzae  A : Hong Kong 1/68 Port Chalmers 1/73 Victoria 3/75 - oeufs embryonnés de 11 jours.

La technique de culture est celle décrite par FAZEKAS (1958). Les egg bits sont maintenus en survie dans les cupules des plaques OMS perspex, avec 0,6 ml de milieu de survie. On les infecte avec 0,1 ml de dilution de virus allant de $10^{-1}$ à $10^{-8}$. Après 6 h. d'incubation à 33°, on inocule une dose de solution de neuraminidase bactérienne à différentes concentrations. On laisse la multiplication se poursuivre pendant 72 h. à 33°. Au bout de ce temps, on prélève 0,1 ml de milieu dans chaque cupule, ce qui permet de comparer la multiplication du virus en fonction des différentes concentrations de neuraminidase bactérienne (d'après le titre hémagglutinant HA et neuraminidase de virus). On réalise aussi une  hémagglutination, directement dans les cupules, avec 0,1 ml de suspension de globules rouges de poule à 0,2%, ce qui permet de comparer le titre infectieux du virus en présence de neuraminidase par rapport au témoin sans neuraminidase bactérienne.

Résultats
---------

Les résultats sont rassemblés dans les tableaux ci-joints n° II à IV.

EXEMPLE VI

- Etude "In Vivo " de l'effet de la neuraminidase bactérienne

A) L'effet inhibiteur de la neuraminidase bactérienne sur la multiplication des virus grippaux, observés "in vitro" , a été testé "in vivo". A cette fin on administre à des lots de furets par voie intranasale, une dose déterminée de virus grippal (MRC 11), puis 6 heures ou 18 h. après, une dose de neuraminidase bactérienne (2mg protéines/ml).

L'effet de la neuraminidase sur la multiplication du virus est suivie en dosant au cours du temps et par comparaison avec des furets témoins ayant reçu uniquement la dose de virus:

. la disparition du virus inoculé

- détermination de son titre hémagglutinant
. neuraminidasique
. infectieux sur oeuf

- dans les écouvillonnages de gorge effectués à différents temps après l'inoculation ( 3 jours, 6 jours, 9 jours, 15 jours).

. la limitation de la production d'anticorps viraux anti-hémagglutine et anti-neuraminidase

- dans les écouvillonnages de gorge par recherche des anticorps locaux
- dans le sérum par recherche des anticorps circulants.

Les lots de furets ayant reçu uniquement le virus grippal, ont manifesté les signes caractéristiques de la grippe: ils ont beaucoup éternué et 6 jours après, ils avaient le nez qui coulait très abondamment, tandis que les furets ayant reçu la neuraminidase bactérienne n'ont pas manifesté ce phénomène.

B) 3 lots de furets sont inoculés par voie intranasale avec une dose de virus MRC 11 (1 ml de mélange allantoïque).

La neuraminidase bactérienne est inoculée, par la même voie, 6 heures après l'injection du virus, à raison d'une dose ( 1 mg de protéines dilué dans 0,5 ml d'eau physiologique).

Des écouvillonages de gorge sont effectués tous les 3 jours après l'inoculation, afin d'établir une cinétique d'absorption et de multiplication du virus au niveau des cellules de la trachée, en présence et en absence de neuraminidase bactérienne.

Matériel et Méthodes

- virus grippal : recombinant MRC 11  (mélange allantoïque)
- neuraminidase bactérienne de Streptococcus pneumoniae
- furets :
. lots témoins recevant le virus
. lots furets traités par le virus + neuraminidase bact.6h.après
. lots furets  traités par le virus + neuraminidase bact. 6h.avant

Les écouvillonnages réalisés tous les 3 jours sont mis en suspension dans 1,5 ml de milieu de survie (Parker) et titrés en hémagglutinine virale (titre HA) et en inhibition d'hémagglutinine ( titre I HA vis-à-vis du virus MRC 11 ).

Une ponction cardiaque légère est effectuée aux 15e et 21e jour après l'inoculation afin de déterminer le taux d'anticorps circulants.

19

Résultats :

Lots témoins : les furets ont été inoculés avec une dose de virus MRC au temps zéro. Puis 6 h. après ont reçu 0,5 ml d'eau physiologique.

3 des lots de furets ont très bien absorbé leur inoculum. Par contre dans un des lots les furets ont beaucoup éternué après l'inoculation.

Tous ces furets ont manifesté d'importants symptômes grippaux dès le 6e jour après l'inoculation :

. yeux très rouges - narines qui coulent - poil terne et hérissé

. tremblements - éternuements - trèsadynamique - perte d'appétit.

On a constaté une mortalité importante pour des lots témoins. Cependant les autres furets témoins ont repris une meilleure santé vers le 9e jour.

Le titre HA qui révèle la présence du virus, augmente jusqu'au 6e jour ( maximum 3e-6e jour ) puis diminue pour disparaître complétement au 15e jour.

Les anticorps anti HA apparaissent au contraire au 6e jour et augmentent progressivement jusqu'au 21e jour.

Lots traités 6h. après l'inoculation du virus : les furets ont été inoculés avec,une dose de virus MRC 11 au temps zéro, puis 6 h. après avec une dose de neuraminidase bactérienne.

Dans l'ensemble, ces furets ont tous un peu éternué après l'inoculation du virus mais ils ont tous très bien absorbé la neuraminidase bactérienne.

Résultats :

20

Les résultats obtenus au cours des différents essais d'administration de la neuraminidase de Streptococcus pneumoniae sont rassemblés dans les tableaux V et VII ci-joints.

EXEMPLE VII

Essai clinique anti-grippal de la neuraminidase de Streptococcus pneumoniae.

A) population étudiée 9 familles ont été concernées par l'essai :

. 19 adultes = 7 hommes âgés de 38 à 66 ans 12 femmes âgées de 35 à 80 ans

. 8 enfants : 3 filles âgées de 10 à 14 ans 5 garçons âgés de 7 à 14 ans

B) Posologie

3 instillations nasales quotidiennes d'une solution aqueuse préssurisée renfermant 330 unités par prise d'essai de neuraminidase de Streptococcus pneumoniae, 2 jours consécutifs, représentant au total 4000 unités de neuraminidase de Streptococcus pneumoniae.

C) Résultats

1) traitement curatif ( 17 malades)

. l'effet subjectif a été très favorable

. une évolution simple, non compliquée de la maladie dans tous les cas

2) traitement préventif (18 malades)

. traitement précoce : un seul sujet en "contact" sur 12 a été malade

. traitement tardif : 3 sujets en "contact" sur 6 ont été malades.

D) Acceptabilité

Excellente dans tous les cas.

EXEMPLE VIII

Vérification de la pureté de la neuraminidase bactérienne de Streptococcus pneumoniae par electrofocalisation.

MODE OPERATOIRE

Préparation des plaques

selon :

- C. KARLSSON, H. DAVIES, J. OHAMN et U.B. ANDERSON (LKB Laboratories applications note 75).

- O. VESTERBERG ( Science Tools, LKB Instrument Journal vol.20, n° 2-3, 1973).

Pour une plaque de 26 x 12,5 cm, on mélange dans l'ordre les solutions suivantes :

- 2,27 g d'acrylamide et 0,073 g de N-N' méthylène bis acrylamide dans de l'eau distillée Q.s.p..........................20 ml

- 7,5 g d'urée dans de l'eau distillée Q.s.p...............36 ml

- Ampholine 3,5-10........................................ 3,2ml

- Riboflavine 0,004 % dans de l'eau distillée.............. 0,8ml


On procède à la photolymérisation.


Préparation des solutions à déposer

La neuraminidase doit avoir une teneur en sels minéraux $\leqslant$ 75 %. Les dépôts effectués doivent être d'environ 350 mcg ( en protéines selon LOWRY) pour la révélation des protéines et de 10.000 U neuraminidase ( unités NANL ) pour la révélation de l' activité neuraminidasique. Ils correspondent à des volumes de solution compris entre 10 et 20 $\mu$ l.


On utilise pour cet essai une neuraminidase d'origine bactérienne titrant 3.700 U/mg et contenant 257 mcg de protéines par milligramme.


On dissout 13 mcg du produit dans 0,1 ml d'eau distillée.


Un prélévement de 20 $\mu$ l sera effectué pour le dépôt correspondant à la révélation de l'activité neuraminidasique ( $\simeq$ 9.620 U

22

neuraminidase). Un prélévement de 10 µl sera effectué pour le dépôt correspondant à la révélation des protéines ( ≃ 334mcg).

Si nécessaire, il sera préparé deux solutions distinctes pour la révélation des protéines et de l'activité neuraminidasique .

## Dépôts

On imprègne deux bandelettes de papier Whatman n°1 de 10 mm x 5 mm; l'une avec la solution pour révélation des protéines et l'autre de solution pour révélation de l'activité neuraminidasique.

On dépose la bandelettes sur le gel dans la zone pH acide (4-5).

## Electrofocalisation

L'électrofocalisation est conduite à + 4°C en cuve LKB réfrigérée. Sous ampérage constant (40 mA) on laisse atteindre 1.000 volts (durée environ 1 h.) . On commute en voltage constant (1.000 volts) jusqu'à ampérage constant (15 mA) . On enlève les bandelettes du gel. On maintient sous 1.000 volts et 15 mA pendant 2h.

La durée totale est d'environ 5 h.

## Détermination du gradient de pH

Il s'effectue à l'aide d'une microélectrode plane (type INGOLD).

## Révélations

## Protéines

La révélation des protéines est réalisée au bleu de Coomassie. ( Selon :
- GRAESSLIN et al. J. Chromatog. 63, 475 (1971)
- O. VESTERBERG Biochim. Biophys. Acta 257, 11 ( 1972)

## Activité neuraminidasique

23

On coule sur le gel une solution de gélose à 1-%, en tampon phosphate de sodium 0,1 M de pH 6,4 contenant 1 mg/ml d'acide méthoxy phényl neuraminique et 1 mg/ml de chlorure du diazonium du 4-amino 2,5-diméthoxy 4'-nitro azobenzène.

On disperse l'acide (méthoxyphényl) neuraminique et le sel de diazonium dans 1 à 2 ml de tampon et on ajoute à la gélose maintenue à 30°C. On maintient quelques minutes à 40°C en atmosphère humide.

Les bandes brunes sur fond marron correspondent aux protéines douées d'activité neuraminidasique.

Après électrofocalisation, sur une même plaque, de deux dépôts de neuraminidase , on révèle les protéines totales de l'un des dépôts au bleu de Coomassie. L'activité neuraminidasique de certaines de ces protéines est révélée à l'aide d'acide méthoxy phényl neuraminique à partir du deuxième dépôt.

On observe ainsi de nombreuses bandes de protéines dont certaines douées d'activité neuraminidasique, les 3 principales en milieu compris entre 6,5 et 9.

Les deux isoenzymes apparaissent dans la zone de pH comprise entre 3,7 et 4,2.

EXEMPLE IX

Exemples de réalisation de formes pharmaceutiques à base de neuraminidase de Streptococcus pneumoniae et étude de leur stabilité.

On effectue une détermination de la stabilité de la neuraminidase bactérienne à diverses températures, à la pulvérisation et à la dispersion par différents générateurs d'aérosol.

L'activité neuraminidasique a été déterminée selon la mé-

24

thode AMINOFF ( Bio. Jour. Vol. 81, p. 384, 1961) les dosages des protéines selon la méthode de LOWRY.

## I - STABILITE A DIVERSES TEMPERATURES

a) d'une solution aqueuse titrant 900 U/ml

neuraminidase bactérienne...................... 250 mg

sérum physiologique à 1 pour 1000 de thiomersal

Q.s.p.................................... 100 ml

| TEMPERATURE DE CONSERVATION | TITRE DE LA SOLUTION T0 | DOSE APRES | | | | | | | | | | | | |
|---|---|---|---|---|---|---|---|---|---|---|---|---|---|---|
| | | 1 Jour | | 2 Jours | | 5 Jours | | 7 Jours | | 9 Jours | | 12 Jours | | 15 Jours | |
| | | Titre | % | Titre | % | Titre | % | Titre | % | Titre | % | Titre | % | Titre | % |
| + 4° C | 900 | 807 | 89,6 | 859 | 95,5 | 816 | 91 | 734 | 81,5 | 844 | 93,7 | 779 | 85,5 | 755 | 94 |
| + 20-22° C | " | 880 | 98 | 835 | 92,7 | 795 | 88,4 | 789 | 87,6 | 807 | 89,6 | 805 | 89,4 | 744 | 82,6 |
| + 37° C | " | 832 | 92,4 | 845 | 94 | 702 | 78 | 664 | 73,7 | 707 | 78,5 | 577 | 64,1 | 443 | 49,7 |

Conclusion : bonne stabilité pour les conditions d'utilisation envisagées ( 5 à 6 jours).

b) d'une solution aqueuse titrant 1700 U/ml

neuraminidase bactérienne..................... 32 mg

sérum physiologique à 1 pour 1000 de thiomersal..

Q.s.p.................................... 55 ml

| TEMPERATURE DE CONSERVATION | TITRE DE LA SOLUTION T0 | DOSE APRES | | | |
|---|---|---|---|---|---|
| | | 5 Jours | | 9 Jours | |
| | | Titre | % | Titre | % |
| 1er essai | 20-22° C | 1 750 | 1 473 | 84,5 | 1 348 | 77 |
| 2e essai | " | 1 717 | 1 450 | 85 | 1 344 | 78,2 |

Conclusion : bonne stabilité pour les conditions d'utilisation envisagées ( 5 à 6 jours).

c) d'une solution aqueuse titrant 2000 U/ml

neuraminidase bactérienne.....................  100 mg

sérum physiologique à 1 pour 25000 de thiomersal..

Q.s.p........................................  100 ml

| TEMPERATURE DE CONSERVATION | TITRE DE LA SOLUTION T0 | DOSE APRES | | | | | |
|---|---|---|---|---|---|---|---|
| | | 5 Jours | | 11 Jours | | 15 Jours | |
| | | Titre | % | Titre | % | Titre | % |
| 20-22° C | 2138 | 1945 | 91 | 2004 | 93,7 | 1934 | 90,4 |

Conclusion : bonne stabilité pour les conditions d'utilisation envisagées ( 5 à 6 jours ).


II- STABILITE A LA PULVERISATION

Solution mise en oeuvre

neuraminidase bactérienne...............  100 mg

sérum physiologique........Q.s.p........  10 ml

| PULVERISATEUR | TITRE DE LA SOLUTION | "PULVERISAT" | |
|---|---|---|---|
| | | Titre * | % |
| 1 * | 2150. | 1630 | 76 |
| 2 * | " | 2056 | 96 |

Moyenne de 18 essais.

Conclusion : la N.ase supporte bien la pulvérisation à l'aide du flacon pulvérisateur muni d'une pompe.


III - STABILITE A LA DISPERSION PAR DIFFERENTS TYPES DE GENERATEURS D'AEROSOL

a) Générateur VAAST

Solution mise en ouvre

neuraminidase bactérienne..................  200 mg

sérum physiologique..........Q.s.p.........  20 ml

| TITRE DE LA SOLUTION MISE EN OEUVRE | AEROSOL | |
|---|---|---|
| | Titre * | % |
| 2 006 * | 1 950 | 97,2 |

Moyenne. de 12 essais.

b) Pression régulée à 0.4 kg/cm$^2$ - solution à 2700 U/ml Solution mise en oeuvre

neuraminidase.................... 20mg

sérum physiologique............. 20ml

| TEMPS DE FONCTIONNEMENT | SOLUTION | | | |
|---|---|---|---|---|
| | Volume | Titre en U/ml | Activité totale | % |
| 0 (départ) | 19 ml | 2 765 | 52 535 | - |
| 2 h. | 10 ml | 4 600 | 46 000 | 87,5 |

N.B. Des témoins ont été effectués avec une solution aqueuse de chlorure de sodium à 10mg/ml et une solution aqueuse de sérum albumine à 1 mg/ml. Après 2 h. de fonctionnement les concentrations des solutions résiduelles (10ml) sont respectivement de 20 mg/ml pour le chlorure de sodium et de 2 mg/ml pour le sérum albumine.

c) Pression régulée à 1,2 kg - Solution à 3600 U/ml Solution mise en oeuvre.

neuraminidase................. 60 mg

sérum physiologique.........; 20 ml

A la. fin de cet essai, l'activité neuraminidasique et la teneur en protéines de la solution résiduelle ont été déterminées.

| TEMPS DE FONCTIONNEMENT | SOLUTION RESIDUELLE | | | | | | | |
| --- | --- | --- | --- | --- | --- | --- | --- | --- |
| | ACTIVITE NEURAMINIDASIQUE | | | | PROTEINES | | | ACTIVITE SPECIFIQUE |
| | Volume en ml | Titre en U/ml | Activité totale | % | mcg/ml | Totales en mg | % | |
| 0 (Départ) | 20 | 3 600 | 72 000 | - | 223 | 4,46 | - | 16 150 |
| 20 minutes | 10 | 4 167 | 41 670 | 58 | 310 | 3,10 | 69,5 | 13 440 |
| 40 minutes | 3 | 5 037 | 15 110 | 21 | 444 | 1,33 | 29,8 | 11 340 |
| 60 minutes | 0 | 1 183 | 2 375 | 3 | 154 | 0,3 | 6,7 | 7 710 |
| | (Rinçage de l'appareil par 2 ml d'eau) | | | | | | | |

d) Générateur '' Colonne JOUAN "

Au cours de ces essais, activité neuraminidasique et teneur en protéines de la solution résiduelle ont été déterminées.

1) Pression régulée à 1 kg - Solution à 3700 U/ml

Solution mise en oeuvre.

neuraminidase bactérienne.......... 60 mg

sérum physiologique............... 20 ml

| TEMPS DE FONCTIONNEMENT | SOLUTION RESIDUELLE | | | | | | | |
| --- | --- | --- | --- | --- | --- | --- | --- | --- |
| | ACTIVITE NEURAMINIDASIQUE | | | | PROTEINES | | | ACTIVITE SPECIFIQUE |
| | Volume en ml | Titre en U/ml | Activité totale | % | mcg/ml | Totales en g | % | |
| 0 (Départ) | 20 | 3 700 | 74 000 | - | 225 | 4,5 | - | 16 400 |
| 1 heure | 8,5 | 6 470 | 55 000 | 73 | 351 | 2,99 | 66,4 | 18 400 |

2) Pression régulée à 0,5kg- Solution à 3700 U/ml

Solution mise en oeuvre.

neuraminidase bactérienne........... 60 mg

sérum physiologiques............... 20 ml

Au cours de ces essais, l'aérosol a été "piégé" par six flacons laveurs montés en série contenant chacun 40 ml d'eau.

Trois éssais ont été effectués. La moyenne des résultats est regroupée dans le tableau ci-après.

| TEMPS DE FONCTIONNEMENT | SOLUTION RESIDUELLE OU SOLUTION DES FLACONS LAVEURS | | | | | | | ACTIVITE SPECIFIQUE |
| | ACTIVITE N.ase | | | | PROTEINES | | | |
| | Volume en ml | Titre en U/ml | Activité totale | % | mcg/ml | Totales en mg | % | |
|---|---|---|---|---|---|---|---|---|
| 0 (Départ) | 20 | 3 700 | 74 000 | – | 224 | 4,48 | – | 16 500 |
| 1 heure | 11,3 | 5 960 | 67 350 | 91 | 283 | 3,25 | 72,6 | 20 700 |
| Flacon laveur 1 | 40 | 63 | 2 720 | | Détermination impossible | – | – | – |
| " " 2 | " | 13,5 | 540 | | " | – | – | – |
| " " 3 | " | 7,1 | 285 | 5,9 | " | – | – | – |
| " " 4 | " | 2,95 | 114 | | " | – | – | – |
| " " 5 | " | 2,6 | 106 | | " | – | – | – |
| " " 6 | " | 15 * | 600 | | " | – | – | – |

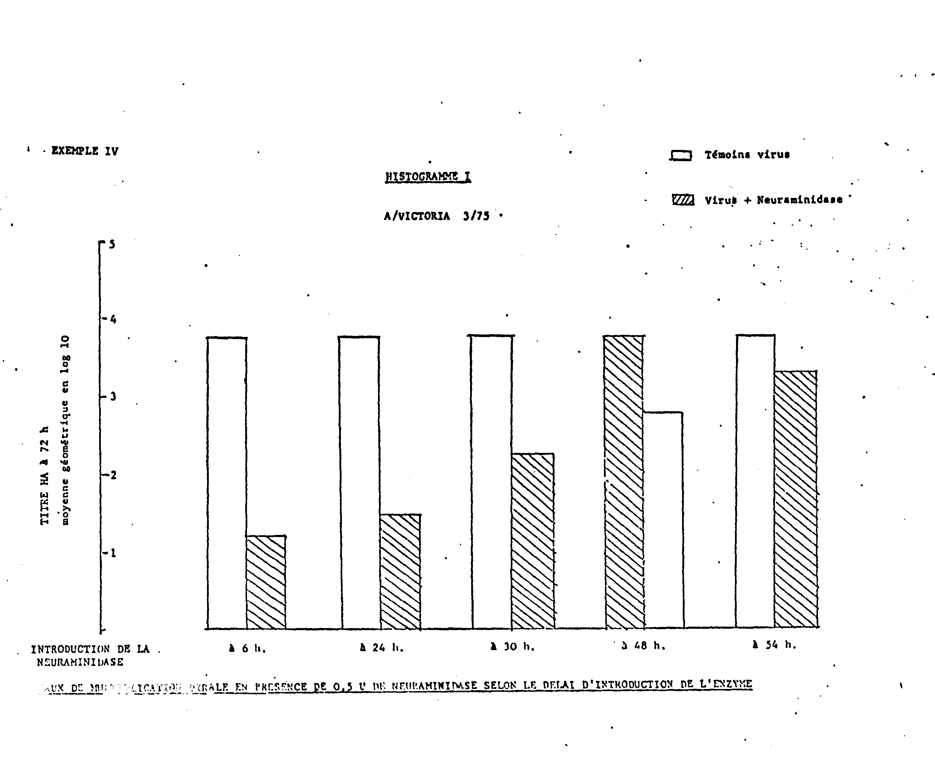

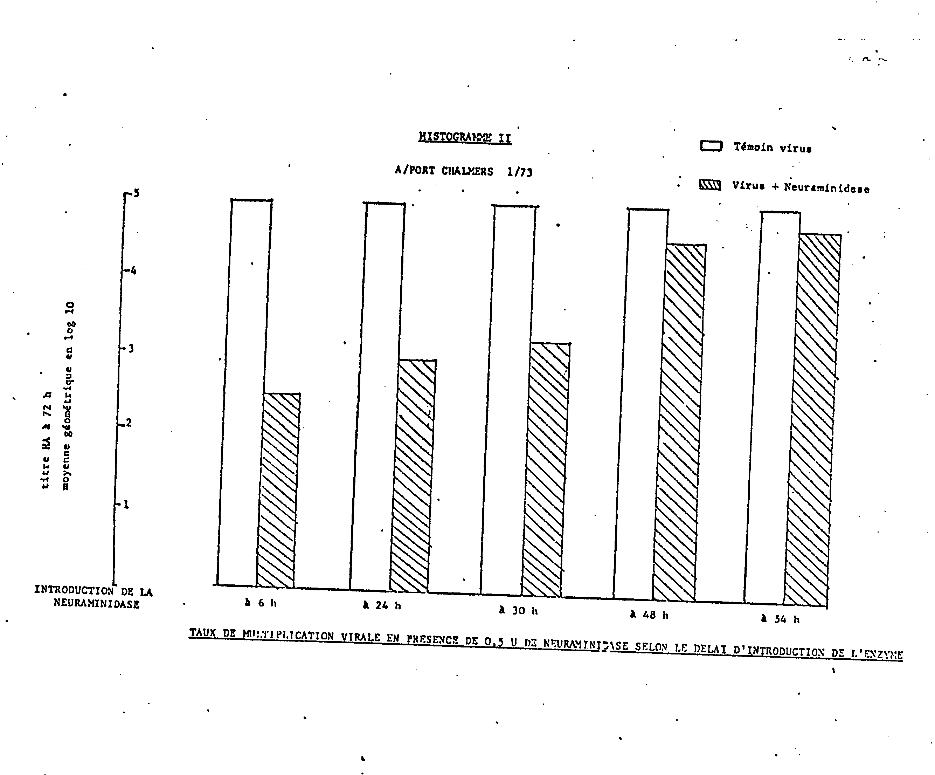

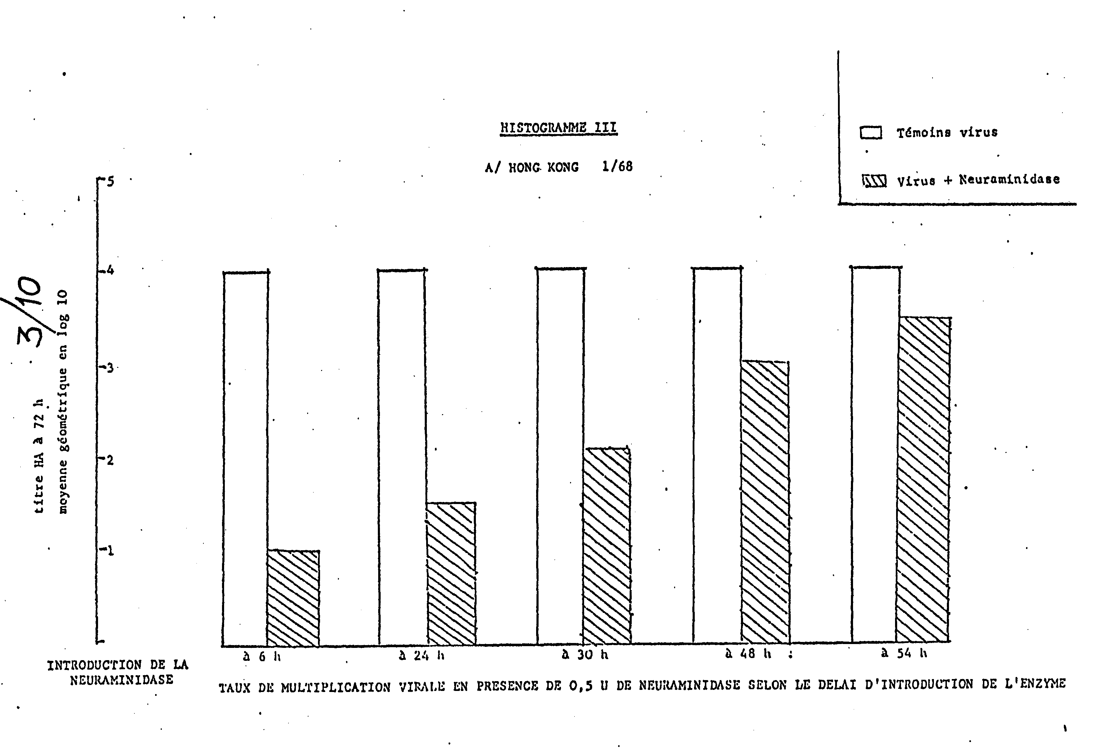

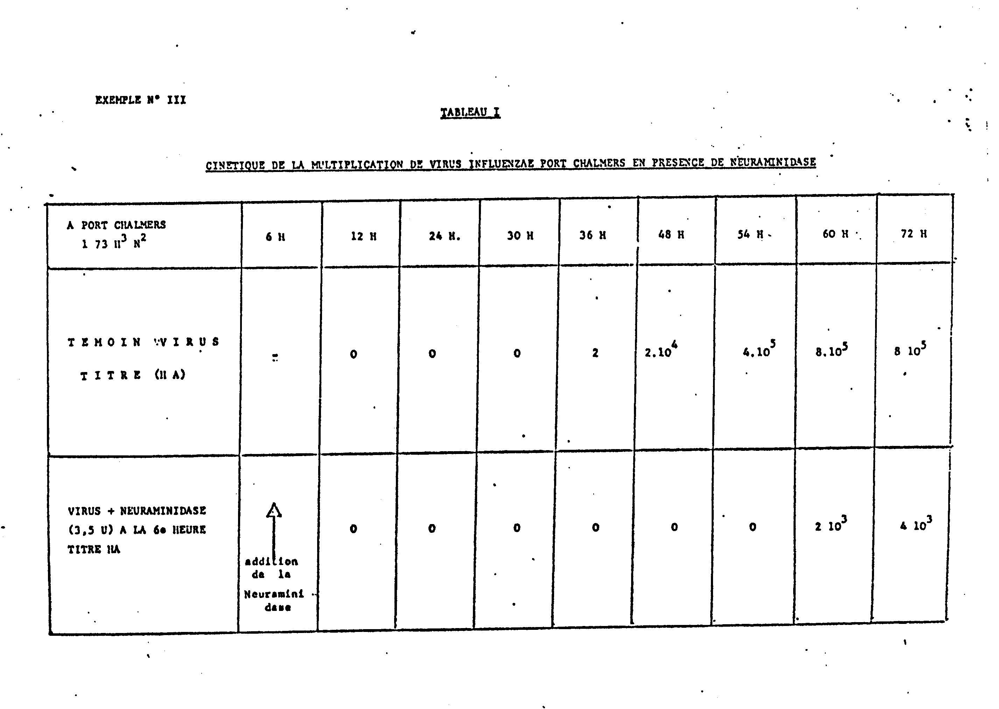

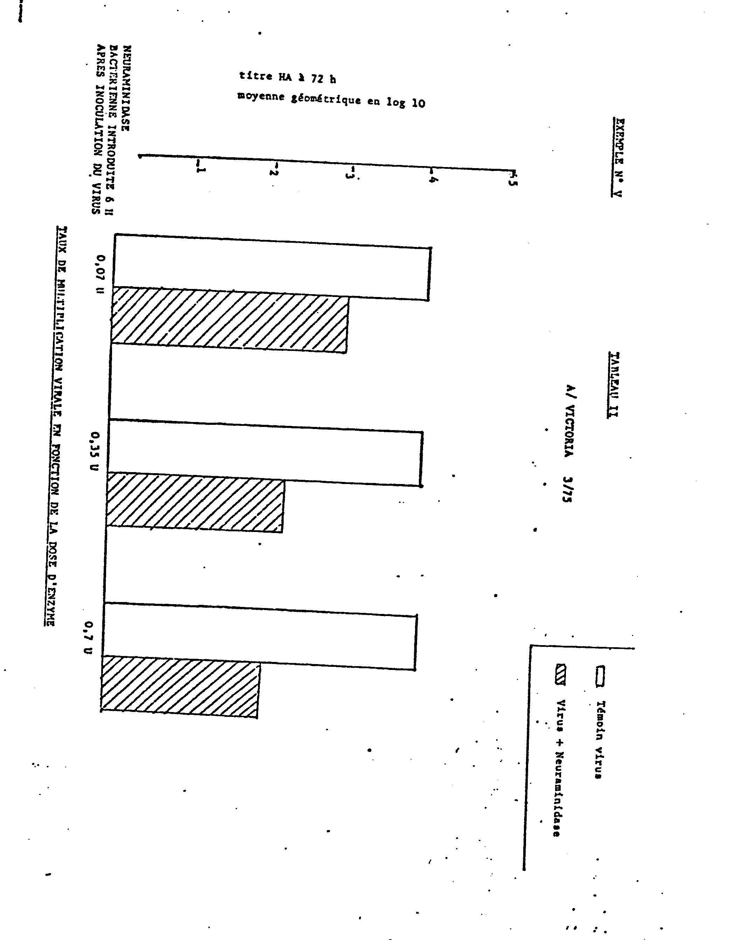

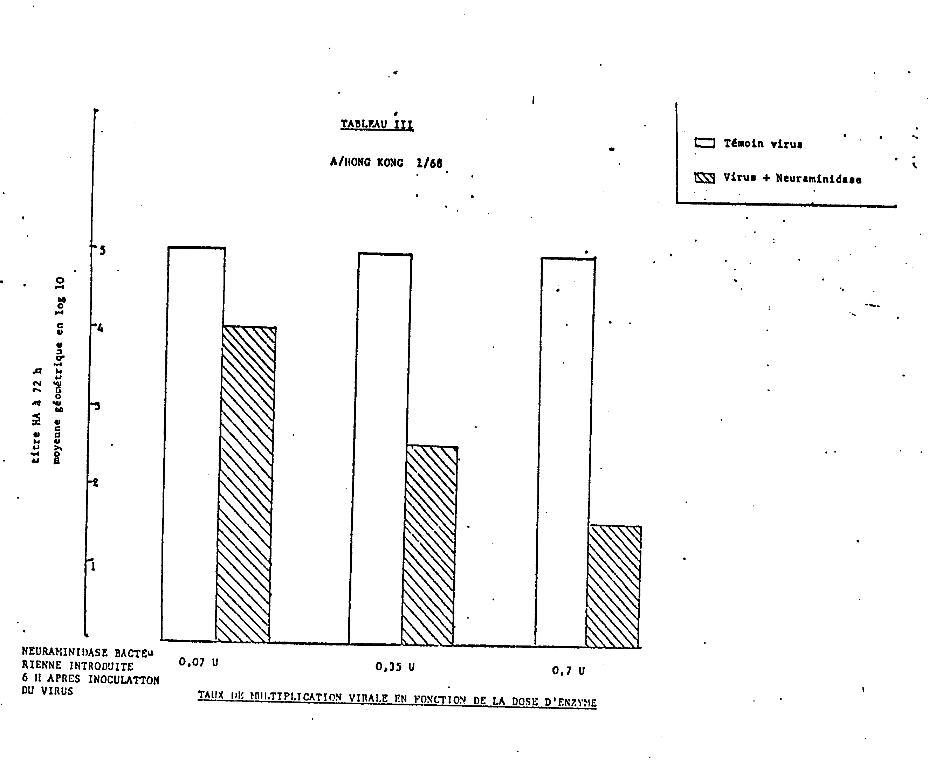

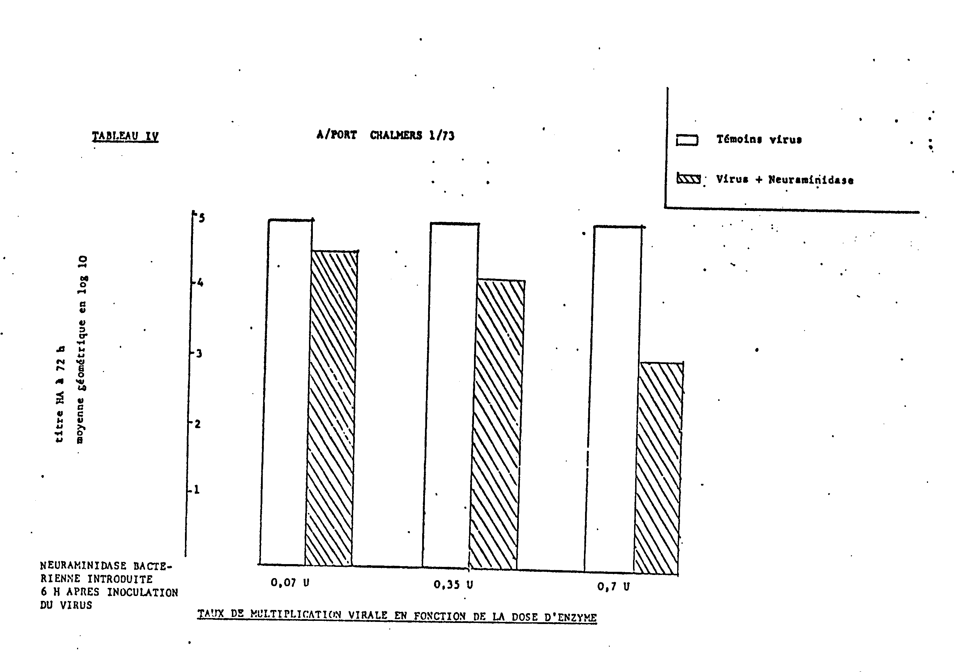

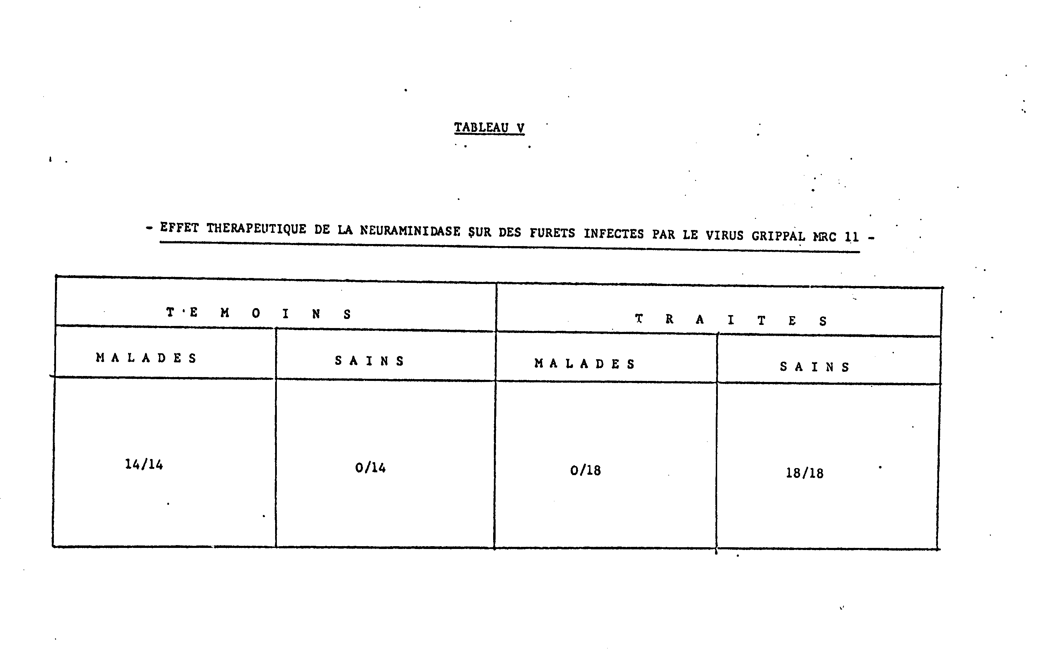

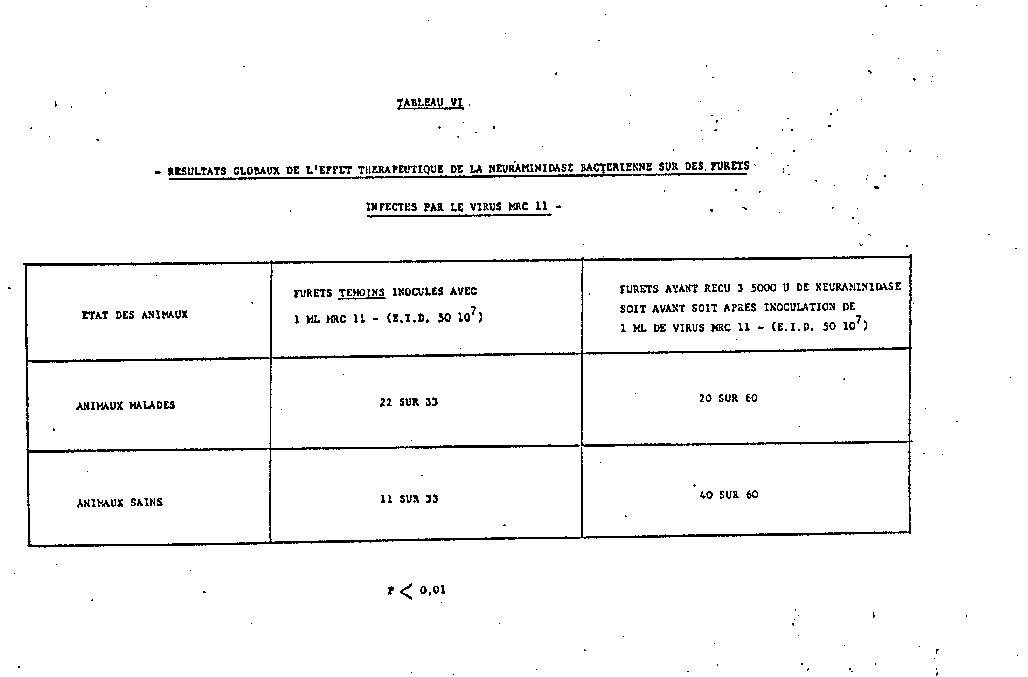

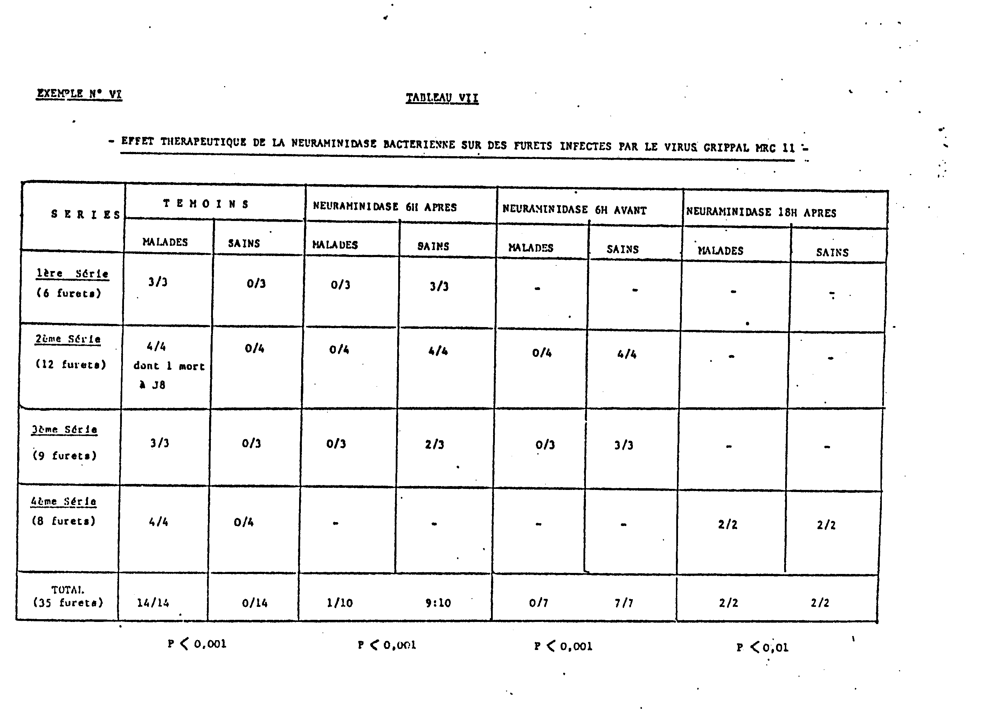

## REVENDICATIONS

1 ) De nouvelles compositions pharmaceutiques destinées au traitement des maladies virales caractérisées en ce qu'elles renferment à titre de principe actif la N-acétyl-neuraminate glycohydrolase obtenue à partir des cultures de Streptococcus pneumoniae ( Micrococcaceae) en association avec un excipient ou un véhicule inerte non-toxique, pharmaceutiquement compatible.

2) Les compositions pharmaceutiques selon la revendication 1, dans lesquelles la N-acétyl neuraminate glycohydrolase est celle obtenue par culture de Streptococcus pneumoniae n° I-044 ( Collection de l'Institut Pasteur de Paris).

3) Les compositions pharmaceutiques selon la revendication 1 ou la revendication 2, dans lesquelles l'excipient ou le véhicule sont adaptés pour l'administration par voie parentérale, permuqueuse, percutanée ou rectale.

4) Les compositions pharmaceutiques selon l'une des revendications 1 à 3 , dans lesquelles la quantité unitaire de N-acétyl-neuraminate glycohydrolase est comprise entre 300 et 5000 U.

EXEMPLE IV

HISTOGRAMME I

A/VICTORIA 3/75

☐ Témoins virus

▨ Virus + Neuraminidase

TITRE HA à 72 h moyenne géométrique en log 10

INTRODUCTION DE LA NEURAMINIDASE

à 6 h.    à 24 h.    à 30 h.    à 48 h.    à 54 h.

TAUX DE MULTIPLICATION VIRALE EN PRESENCE DE 0,5 U DE NEURAMINIDASE SELON LE DELAI D'INTRODUCTION DE L'ENZYME

0004214

HISTOGRAMME II

A/PORT CHALMERS  1/73

▭ Témoin virus

▨ Virus + Neuraminidase

titre HA à 72 h
moyenne géométrique en log 10

INTRODUCTION DE LA
NEURAMINIDASE

à 6 h     à 24 h     à 30 h     à 48 h     à 54 h

TAUX DE MULTIPLICATION VIRALE EN PRESENCE DE 0,5 U DE NEURAMINIDASE SELON LE DELAI D'INTRODUCTION DE L'ENZYME

HISTOGRAMME III

A/ HONG KONG 1/68

☐ Témoins virus

▨ Virus + Neuraminidase

0004214

3/10

titre HA à 72 h moyenne géométrique en log 10

INTRODUCTION DE LA NEURAMINIDASE

à 6 h    à 24 h    à 30 h    à 48 h :    à 54 h

TAUX DE MULTIPLICATION VIRALE EN PRESENCE DE 0,5 U DE NEURAMINIDASE SELON LE DELAI D'INTRODUCTION DE L'ENZYME

4/10

EXEMPLE N° III

TABLEAU I

CINETIQUE DE LA MULTIPLICATION DE VIRUS INFLUENZAE PORT CHALMERS EN PRESENCE DE NEURAMINIDASE

| A PORT CHALMERS $1\ 73\ H^3\ N^2$ | 6 H | 12 H | 24 H. | 30 H | 36 H | 48 H | 54 H | 60 H | 72 H |
|---|---|---|---|---|---|---|---|---|---|
| TEMOIN VIRUS TITRE (HA) | = | 0 | 0 | 0 | 2 | $2.10^4$ | $4.10^5$ | $8.10^5$ | $8\ 10^5$ |
| VIRUS + NEURAMINIDASE (3,5 U) A LA 6e HEURE TITRE HA | addition de la Neuraminidase | 0 | 0 | 0 | 0 | 0 | 0 | $2\ 10^3$ | $4\ 10^3$ |

EXEMPLE N° V                    TABLEAU II

                            A/ VICTORIA   3/75

| | Témoin virus |
| | Virus + Neuraminidase |

titre HA à 72 h
moyenne géométrique en log 10

NEURAMINIDASE
BACTERIENNE INTRODUITE 6 H
APRES INOCULATION DU VIRUS

0,07 U              0,35 U              0,7 U

TAUX DE MULTIPLICATION VIRALE EN FONCTION DE LA DOSE D'ENZYME

titre HA à 72 h

moyenne géométrique en log 10

**TABLEAU III**

A/HONG KONG 1/68

NEURAMINIDASE BACTE-
RIENNE INTRODUITE
6 H APRES INOCULATION
DU VIRUS

TAUX DE MULTIPLICATION VIRALE EN FONCTION DE LA DOSE D'ENZYME

0,07 U

0,35 U

0,7 U

☐ Témoin virus

▨ Virus + Neuraminidase

TABLEAU IV        A/PORT CHALMERS 1/73

□ Témoins virus

▨ : Virus + Neuraminidase

titre HA à 72 h
moyenne géométrique en log 10

NEURAMINIDASE BACTE-
RIENNE INTRODUITE
6 H APRES INOCULATION
DU VIRUS

0,07 U      0,35 U      0,7 U

TAUX DE MULTIPLICATION VIRALE EN FONCTION DE LA DOSE D'ENZYME

TABLEAU V

- EFFET THERAPEUTIQUE DE LA NEURAMINIDASE SUR DES FURETS INFECTES PAR LE VIRUS GRIPPAL MRC 11 -

| TEMOINS | | TRAITES | |
|---------|---------|---------|---------|
| MALADES | SAINS | MALADES | SAINS |
| 14/14 | 0/14 | 0/18 | 18/18 |

0004214

TABLEAU VI

- RESULTATS GLOBAUX DE L'EFFET THERAPEUTIQUE DE LA NEURAMINIDASE BACTERIENNE SUR DES FURETS

INFECTES PAR LE VIRUS MRC 11 -

| ETAT DES ANIMAUX | FURETS TEMOINS INOCULES AVEC 1 ML MRC 11 - (E.I.D. 50 $10^7$) | FURETS AYANT RECU 3 5000 U DE NEURAMINIDASE SOIT AVANT SOIT APRES INOCULATION DE 1 ML DE VIRUS MRC 11 - (E.I.D. 50 $10^7$) |
|---|---|---|
| ANIMAUX MALADES | 22 SUR 33 | 20 SUR 60 |
| ANIMAUX SAINS | 11 SUR 33 | 40 SUR 60 |

$P < 0,01$

EXEMPLE N° VI                                                        TABLEAU VII

- EFFET THERAPEUTIQUE DE LA NEURAMINIDASE BACTERIENNE SUR DES FURETS INFECTES PAR LE VIRUS GRIPPAL MRC 11 -

10/10

| SERIES | TEMOINS | | NEURAMINIDASE 6H APRES | | NEURAMINIDASE 6H AVANT | | NEURAMINIDASE 18H APRES | |
|---|---|---|---|---|---|---|---|---|
| | MALADES | SAINS | MALADES | SAINS | MALADES | SAINS | MALADES | SAINS |
| 1ère Série (6 furets) | 3/3 | 0/3 | 0/3 | 3/3 | - | - | - | - |
| 2ème Série (12 furets) | 4/4 dont 1 mort à J8 | 0/4 | 0/4 | 4/4 | 0/4 | 4/4 | - | - |
| 3ème Série (9 furets) | 3/3 | 0/3 | 0/3 | 2/3 | 0/3 | 3/3 | - | - |
| 4ème Série (8 furets) | 4/4 | 0/4 | - | - | - | - | 2/2 | 2/2 |
| TOTAL (35 furets) | 14/14 | 0/14 | 1/10 | 9:10 | 0/7 | 7/7 | 2/2 | 2/2 |
| | $P < 0,001$ | | $P < 0,001$ | | $P < 0,001$ | | $P < 0,01$ | |

# EUROPEAN SEARCH REPORT

European Patent Office

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| | FR - A - 2 350 847 (BEHRINGWERKE) <br>    * Revendications 1-3; page 1, lignes 19-22; page 2, lignes 14-23 * <br><br> -- <br><br> FR - A - 2 262 962 (SCIENCE UNION. & CIE) <br>    * Revendications 3 et 8 * <br><br> -- | 1-4 <br><br><br><br><br> 1-4 | A 61 K 37/54// <br> C 12 D 13/10 |
| D | C.R. ACAD. Sc. Paris, Série D, tome 281, 1545-47, 17 novembre 1975; <br> PATRICE BINDER et al.: "Etude, chez le rat, des effets de la neuraminidase, bactérienne et virale, en injection intratumorale" <br>    * Article en entier * <br><br> -- <br><br> CHEMICAL ABSTRACTS, vol. 83, no. 25, december 22, 1975, ref. 204534a Columbus, Ohio, USA <br> E. PUCHELLE et al.: "Effect of neuraminidases from Diplococcus pneumoniae and Clostridium perfringens on the viscoelastic properties of bronchial secretions" <br> & Biorheology 1975, 12(3-4),219-24 <br>    * Résumé * <br><br> -- <br><br> CHEMICAL ABSTRACTS, vol. 79, 121829s (1973) no. 21, november 26, 1973, page 15 Columbus, Ohio, USA <br> M.A. Madoff et al.: "Neuraminidase-induced delay of mouse skin graft | 1-4 <br><br><br><br><br><br><br><br><br> 1-4 <br><br><br><br><br><br><br><br><br><br><br> 1-4 | |

**TECHNICAL FIELDS SEARCHED (Int.Cl.²)**

A 61 K 37/54
C 12 D 13/10

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| La Haye | 06-06-1979 | RIJCKEBOSCH |

EPO Form 1503.1 06.78

0004214

European Patent
Office

EUROPEAN SEARCH REPORT

Application number

EP 79 40 0094

-2-

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | rejection. <br><br>& Transplantation 1973 16(2), 157-60 <br><br>  * Résumé * <br><br>-- <br><br>CHEMICAL ABSTRACTS, Vol. 79, no. 19, november 12, 1973, 112105a Columbus, Ohio, USA Z. BENTWICH "Sheep red cell binding to human lymphocytes treated with neuraminidase. Enhancement of T cell binding and  identification of a subpopulation of B cells" page 61 <br><br>& J. Exp. Med. 1973, 137(6) 1532-8 <br><br>  * Résumé * <br><br>---- | 1-4 | |
| | | | TECHNICAL FIELDS SEARCHED (Int. Cl.²) |